(19) Europäisches Patentamt
European Patent Office
Office européen des brevets

(11) Publication number: **0 043 274**
**B1**

(12) **EUROPEAN PATENT SPECIFICATION**

(45) Date of publication of patent specification: **16.01.85**

(21) Application number: **81302950.1**

(22) Date of filing: **29.06.81**

(51) Int. Cl.⁴: **A 61 K 33/04,** A 61 K 31/13, A 61 K 31/185, C 07 C 87/06, C 07 C 87/30

# (54) Antithrombotic treatment.

(30) Priority: **30.06.80 US 164303**
**22.12.80 US 218412**
**22.12.80 US 218414**
**16.06.81 US 271848**
**16.06.81 US 271849**

(43) Date of publication of application:
**06.01.82 Bulletin 82/01**

(45) Publication of the grant of the patent:
**16.01.85 Bulletin 85/03**

(84) Designated Contracting States:
**AT BE CH DE FR GB IT LI LU NL SE**

(56) References cited:
**FR-M- 2 613**
**GB-A- 816 592**
**US-A-3 279 993**
**US-A-3 906 109**

**DICTIONNAIRE VIDAL, 1968, O.V.P., Paris, FR**
**DICTIONNAIRE VIDAL, 1979, O.V.P., Paris, FR**
**DICTIONNAIRE VIDAL, 1971, O.V.P., Paris, FR**

(73) Proprietor: **T AND R CHEMICALS, INC.**
**700 Celum Road**
**Clint Texas 79836 (US)**

(72) Inventor: **Arias Alvarez, José Antonio**
**Carpatos 705**
**Mexico 10 D.F. Mexico (MX)**
Inventor: **Thompson, Ralph B.**
**35S74 Adams**
**Oak Brook Illinois 60521 (US)**

(74) Representative: **Burford, Anthony Frederick et al**
**W.H. Beck, Greener & Co. 7 Stone Buildings**
**Lincoln's Inn**
**London WC2A 3SZ (GB)**

(56) References cited:
**ROTE LISTE, 1979, Cantor, Aulendorf/Württ., DE**
**UNLISTED DRUGS, vol. 27, September 1975, Chatham, N.J. US**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 043 274 B1

Courier Press, Leamington Spa, England.

**Description**

Sodium bisulphite (usually shown by the formula $NaHSO_3$) has previously been used for many commerical purposes, for example, as a preservative, for preventing deterioration of liquids, such as foodstuffs and pharmaceutical solids, and it has been used medically, externally for the treatment of parasitic skin diseases and internally as a gastrointestinal antiseptic.

The solid sodium bisulphite of commerce reportedly consists chiefly of sodium metabisulphite, $Na_2S_2O_5$, and sodium acid sulphite, $NaHSO_3$, and, for the purposes of this invention, it is believed to possess the same properties as (and to be equivalent to) sodium bisulphite, when in the form of an aqueous solution.

French Patent No. 2613 M (and corresponding UK Patent No. 1033378) disclose the use of formaldehyde bisulphites to stabilize protamine in injectable solutions. Protamine is a generic term designating a class of low molecular weight proteins having mild anticoagulant properties but principally of use as an antidote to heparin. There is no reference to any antithrombotic or related activity of the formaldehyde bisulphites.

Reiss and Gerstl (Exptal. Med. Surg. Vop. 4, 1946, 338—347) disclose the injection into rabbits of ethanolamine bisulphite 0.6M aqueous solution when comparing the systemic effects of thioglycolic acid and sodium bisulphite. There is no reference to any antithrombotic activity of either of said bisulphites.

Anticoagulants and antithrombotic agents are a group of compounds with diversified pharmacological actions which are used in a variety of clinical thrombotic disorders. Thrombotic disorders are generally divided into venous thrombosis and arterial occlusive disorders. Venous thrombosis of the lower extremities is important, because it can cause pulmonary embolisms which may be fatal. "Heparin" and "Warfarin" are commonly used in clinical medicine for the prevention and treatment of deep venous thromboses and pulmonary embolisms. Their main pharmacological actions are to inhibit or interrupt blood coagulation activity.

Platelets play an important part in arterial thrombosis. Drugs which inihibit platelet aggregation are generally regarded as potentially useful for the prophylactic therapy of arterial thrombotic disorders, including, for example, strokes, myocardial infarctions and peripheral vascular disease. Despite the availability of many agents which possess platelet anti-aggregatory properties, only a few are currently under clinical trial (for example, "Aspirin"®, dipyridamole and sulphinpyrazone). None of these agents exhibits unequivocal efficacy. Compounds with more specific pharmacological actions are urgently sought in order to provide better medical care for patients with these serious disorders.

An anticoagulant is a substance which inhibits coagulation of the blood. A platelet anti-aggregatory agent is a substance which inihibits platelet aggregation.

An antithrombotic agent is a substance which inhibits the formation or development of a thrombus (or thrombosis). For present purposes, the term "thrombus" or its equivalent includes the term "embolus", unless otherwise specifically indicated. In general, an antithrombotic agent, in the presence of mammalian blood or appropriately-prepared plasma, may display anticoagulant activity and/or platelet anti-aggregatory activity.

A class of active agents has now been discovered, the members of which, when orally ingested and/or injected, produce amelioration of a thrombotic condition in mammals (including man), when used in antithrombotically-effective amounts as described below.

According to one aspect of this invention, a pharmaceutical composition is provided for use in treating an actual or incipient thrombotic condition in a mammal, and is characterized in that it contains at least one carrier substance and, as the active treating agent, at least one compound selected from the following compounds:

(A) compounds of formula:

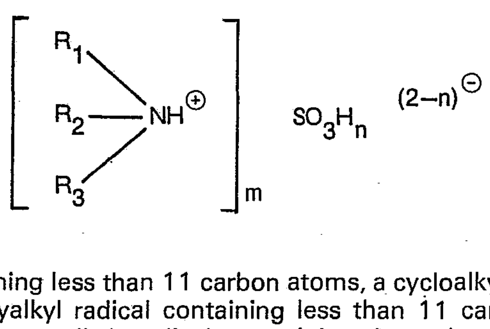

wherein:

$R_1$ is an alkyl radical containing less than 11 carbon atoms, a cycloalkyl radical containing 6 to 10 carbon atoms or a monohydroxyalkyl radical containing less than 11 carbon atoms,

$R_2$ is a hydrogen atom, an alkyl radical containing less than 11 carbon atoms or a monohydroxyalkyl radical containing less than 11 carbon atoms, or

$R_1$ and $R_2$ together form an unsubstituted morpholine, piperidine or hexamethyleneimine ring or such a ring carrying on one of its carbon atoms an alkyl radical containing less than 11 carbon atoms,

$R_3$ is a hydrogen atom, an alkyl radical containing less than 11 carbon atoms, a monohydroxyalkyl radical containing less than 11 carbon atoms or a radical of the formula:

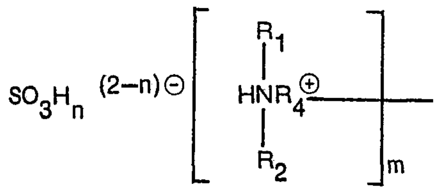

wherein:

$R_1$ and $R_2$ have the meanings defined above,

$R_4$ is a divalent saturated aliphatic radical containing less than 11 carbon atoms, and m is 1 or 2, n is 0 or 1 and the sum of m and n is 2;

(B) compounds of either of the formulae:

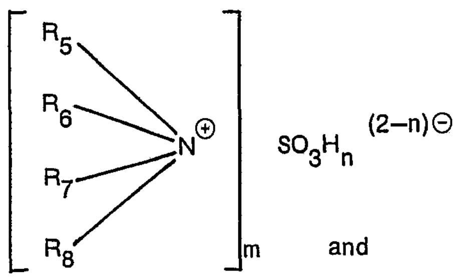

and

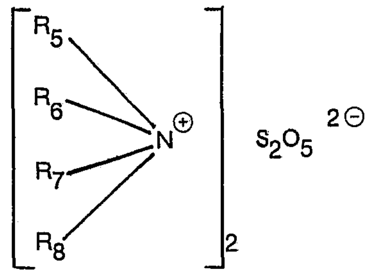

wherein:

$R_5$, $R_6$, $R_7$ and $R_8$ are the same or different and each is an alkyl radical containing less than 11 carbon atoms, and

m and n have the meanings defined above;

(C) compounds of the formula:

$$R—CHOH—SO_3M$$

wherein:

R is a hydrogen atom or a hydroxymethyl radical and

M is an alkali metal or an ammonium group, but excluding:—

(a) 0.6M injectable aqueous solutions of ethanolamine bisulphite, and

(b) compositions containing a formaldehyde bisulphite and a protamine.

Examples of suitable alkylamine starting materials for making compounds of the formula in (A) include methylamine, dimethylamine, trimethylamine, ethylamine, tripentylamine, monocyclohexylamine, dimethylcyclohexylamine, dimethyl-ethylamine, dimethylcyclohexylamine and tri-*n*-butylamine. Trimethylamine salts tend to be undesirable because of a strong associated odour, however with this sole exception, tri(lower alkyl)amines are one currently preferred class of starting materials for the preparation of compounds of the formula in (A). Another currently preferred class of starting materials for such a preparation comprises monoalkylamines having 5 to 10 carbon atoms per molecule, such as mono-*n*-octylamine. Examples of suitable diamine starting materials for making compounds of the formula in (A) include ethylene-diamine, hexamethylene-diamine, 1,2-propylenediamine, tetramethyl-ethylene-diamine and $N_1$, $N_2$-dimethyl-ethylene-diamine.

Examples of quaternary ammonium bisulphite compounds include tetramethyl-ammonium-bisulphite, tetraethyl-ammonium-bisulphite, tetrabutyl-ammonium-bisulphite, trimethylethyl-ammonium-bisulphite and tetramethyl-ammonium-metabisulphite. Tetra-(lower alkyl)-ammonium-sulphites are also active agents, however.

Antithrombotic agents of this invention are believed to be usable in both arterial thrombosis and venous thrombosis.

Examples of clinical thrombotic conditions include stroke (as a cerebral vascular thrombosis), myocardial infarction (coronary artery disease), peripheral vascular disease, cardiac valve replacement, deep vein thrombosis and pulmonary embolism.

The mechanism(s) by which the active agents function is currently unknown; however, an inhibition of platelet aggregation and a prolongation of normal blood coagulation time appear to be associated with their use in the manner disclosed herein.

In one aspect, the present invention is concerned with certain organic bisulphite and sulphite pharmaceutical compositions for use as antithrombotic, anticoagulant and platelet anti-aggregatory agents in mammalian medicine (including human medicine).

In another aspect, compositions of the present invention can be used for the control of and/or the

prevention of an embolus or a thrombus in man by oral ingestion and/or by injection of a pharmaceutically-effective amount of one or more compounds comprising the active agents of this invention.

In another aspect, the compositions of the present invention can provide symptomatic and objective improvement in thrombotic (including cardiovascular) disease conditions, for example, abnormal coagulation or intravascular thrombosis, in man. The term "symptomatic improvement", as used herein, means an improvement in a patient's subjective symptoms (e.g., as reported by the patient). The term "objective improvement", as used herein, means a measurable change in a patient's condition.

More particularly, the compositions of this invention can be used in a process for treating a human or other mammal wherein there is introduced orally and/or by injection into such mammal a pharmaceutically-effective amount of an active agent of this invention as an antithrombotic agent.

Sulphite and/or bisulphite anions do not normally occur in human tissues or blood, so far as is now known.

In medicine, arterial thrombosis is diagnosable, for example, by clinical manifestations by arteriography and, recently, by an indium 111 platelet labelling technique (see, for example, the article entitled "Differential Effects of Two Doses of Aspirin on Platelet-Vessel Wall Interaction In Vivo" by K. K. Wu et al being published in the Journal of Clinical Investigation August, 1981).

Also, a thrombosis is detectable, for example, from a patient's conditions symptomatically perceivable by a skilled medical practitioner and well known to the art of medicine. Objectively, various methods are available, including venography, impedance plethysmography, doppler ultrasound and the $^{125}$I-fibrinogen test (see, for example, the articles by Kakkar, "Archives of Surgery", 104, page 152 (1972) and by Kelton, J. G. et al, Journal of Clinical Investigation, Vol. 62, pgs. 892—895, (1978).

The present invention does not contemplate feeding a normal patient (that is, one not suffering from a thrombotic condition) an active agent of this invention at a pharmaceutically effective dosage as indicated herein.

The term "thrombotic condition" as used herein, means both:

(a) an existing thrombus (including an embolus);

(b) an incipient thrombus (including an incipient embolus).

An "incipient thrombus" or "incipient thrombotic condition", as used herein, is a condition which can exist in a patient who is predisposed to the development of a thrombotic condition. For example, diabetes mellitus and hyperlipidemia are conditions which predispose a patient to arterial thrombosis. On the other hand, surgery, trauma and bed rest, for example, predispose a patient to venous thrombosis.

Those skilled in the practice of medicine routinely determine the presence of a thrombotic condition (including an actual thrombus in a patient).

Preferably, the practice of this invention *in vivo* involves introducing into the blood of a patient, such as a human, the equivalent of 1 to 100 milligrams per kilogram of mammal body weight (including human) per day, though larger or smaller dosage rates may be employed, if desired, within the scope of this invention. The exact amount or dose in any given case is selected to be sufficient and appropriate for achieving a desired antithrombotic effect.

In general such an introduction may be commenced at a dosage rate within the range above indicated as soon as a thrombotic condition (or a thrombus) is found in a patient.

For example, it is preferred that as a first step, a determination is made that a patient suffers from a thrombotic condition. Then, one starts orally feeding and/or injecting such patient with at least one active agent of the present invention at an effective dosage rate in the range indicated above. Currently, an especially preferred dosage rate is 20 to 50 mg/kg per day. Preferably, at least two or three spaced doses per day are given, each such dose being conveniently administered around a meal-time. Any convenient dosage arrangement can be employed.

Not uncommonly, it is desirable or necessary to start treatment immediately upon the discovery of a patient's thrombotic condition, in order to avoid damage or injury or perhaps even death of the patient, as from an embolus. If oral administration is not convenient or rapid enough, the active agent can be directly introduced by injection into the patient, if desired, such as intravenously, intramuscularly or subcutaneously. When an active agent is directly introduced, it is preferably dissolved in an aqueous medium, the total amount of active agent introduced into such medium preferably being within the range from 1 to 11 weight percent (based on the total solution weight). Distilled water is preferred as the aqueous medium. If desired, conventional (standardized) aqueous media can be used as vehicles for such introduction; for example, standard saline solutions can be used as vehicles.

In order to evaluate an antithrombotic effect it is preferable to withdraw samples of blood from a patient undergoing treatment and measure platelet aggregation. One method is described by Born in Nature 194, pp. 927—929 (1962) and may be used for this purpose, if desired.

After administration has started, the dosage rate is preferably adjusted to a value which is sufficient to disrupt platelet function and/or coagulation factors and thereby achieve a desired antithrombotic effect.

An active agent of this invention is characteristically capable of exhibiting platelet aggregation both *in vitro* and *in vivo*. Also, such an active agent is characteristically capable of lengthening both PT (prothrombin time) and PTT (blood partial thromboplastin time) *in vitro*. The dosage rate of the active agent is currently believed to be related to the resulting effects upon blood factors, such as inhibition of platelet aggregation. Consequently, under this preferred procedure, use of an active agent at a suitable dose for an individual patient ameliorates that patient's thrombotic condition.

Selected blood parameters of a patient are preferably determined before dosing with an active agent is started, when time permits. Preferably, dosage rate adjustment is made while administration of an active agent is continuing. The amount of adjustment (or incremental change in dosage) is determinable by comparing a patient's measured values during administration of active agent to desired values (such as the patient's own corresponding starting values or normal species, e.g. human, values). Inhibition of platelet aggregation can be used for such measurements. Then, the deviation, if any, from the patient's measured values is compared to the desired values (the patient's starting values or normal species values, for example). Then a change in dosage rate may be made to correct any deviation so determined.

For instance, in humans, normal values for platelet aggregation are dependent upon the particular agent used for stimulation. For example, when adenosine diphosphate (ADP) at 3 micromolar concentration is employed, platelet aggregation values fall typically in the range from 50% to 100% of light transmission. Other stimulation agents include collagen, epinephrine and arachidonic acid.

Also, in humans, normal PT values fall in the range from 11 to 13 seconds, while normal PTT values fall in the range from 25 to 41 seconds. If PT values and/or PTT values could be measured in a given patient, for the purpose of achieving a desired antithrombotic effectiveness, it is currently estimated that a lengthening of PTT value to 1.5 to 2 times a PTT value in such normal range in a given starting patient is appropriate or suitable for antithrombotic effectiveness, which amounts to a PTT value for a given patient of 45 to 60 seconds; such an estimate is consistent, for example, with the lengthened PTT values achieved in the human use of heparin, sometimes employed previously as an antithrombotic agent. Similarly, it is currently estimated that a lengthening of PT value to 2.0 times a PT value in such normal range in a given starting patient is appropriate or suitable for antithrombotic effectiveness, which amounts to a PT value for a given patient of 22 to 26 seconds; such an estimate is consistent, for example, with the lengthened PT values achieved in the human use of coumadin (warfarin), sometimes employed previously as an antithrombotic agent. The active agents of the present invention, contrary to such prior art agents, surprisingly appear to affect both PT and PTT values. The mechanism by which the present active agents work is apparently substantially different from, and not comparable to, the prior art agents. Study and evaluation of the active agents of this invention continues.

Contrary to prior agents (such as heparin and coudamin), the active agents of the present invention appear to affect both blood coagulation factors and platelet aggregation. Conveniently and preferably, measurements of blood factors are carried out periodically, such as every 3 to 7 days, on a patient undergoing treatment under the practice of this invention.

An active agent can be given orally, in the form of a capsule or tablet, or in the form of a solution (e.g. aqueous). Also, an active agent can be injected in the form of an aqueous solution.

A particularly preferred antithrombotic field of use is in post-operative treatment, as when arteries or deep veins may be involved in, or threatened by, a thrombotic condition.

By way of explanation, as those familiar with mammalian anatomy appreciate, the venous system in the lower extremities consists of superficial and deep veins. Because of the manner in which the deep veins interconnect and supply blood to the heart and lungs, a thrombus occurring in the deep veins, but not in the superficial veins, can become the source of a blood clot which is moved through the veins and becomes lodged in the lungs, resulting in a pulmonary embolus, which can have obvious catastrophic effects (including causing death). Examples of deep veins include the iliac, the femoral, the popliteal and the calf veins. The prevention of pulmonary emboli following surgery affecting the deep veins in the lower extremities is a significant medical problem. One solution to this problem is to prevent thrombi from occurring and/or developing in deep veins. To achieve this, active agents of this invention appear to be well suited. Thus, in carrying out this invention, one may achieve a symptomatic and objective improvement of a deep vein thrombotic complication in a patient during post-operative care, inhibiting intravascular thrombus formation (including embolism).

In one preferred practice of this invention an aqueous solution of 1 to 10 percent by weight of an active agent of this invention is prepared. Then, this solution is orally consumed by a human or is injected at a total (or accumulated) dosage rate ranging from 1.0 to 50 mg per kg of body weight per day, more preferably in the form of at least two spaced doses per day, and still more preferably in the form of at least three spaced doses per day, each dose preferably being taken around a meal-time. Instead, solid or encapsulated active agents may be orally consumed.

Because of a tendency for bisulphites to undergo oxidation when in aqueous solution, it is currently preferred to minimize contact of active agents with oxygen before use. However, the compounds of the formula in (C) appear to be stable towards oxygen.

The water used in such a solution is preferably purified (e.g., filtered, deionized or distilled). After preparation, the solution is preferably stored in a closed container to reduce oxidation.

Such an aqueous solution can be directly used in carrying out this invention, in which case such a solution can be dispensed dropwise or it can be encapsulated, for instance, and used as measured dosage units, as desired. For example, an aqueous solution can be injected into a patient, or it can be directly consumed by a patient as drops (e.g., from 5 to 9 drops per meal for each of the two or three meals eaten by such patient per day, depending upon an individual patient's body weight).

Symptomatic improvement in varicose veins and in haemorrhoids may be observable when using an active agent of this invention.

Compounds which are active agents of the formulae in (A) and (B) are conveniently prepared by preparing an aqueous solution (preferably using purified or distilled water) of a corresponding lower (or corresponding quaternary ammonium hyroxide) alkylamine compound, present at a specified or calculated concentration, such as 10 weight percent. Then, $SO_2$ gas is bubbled through this solution until the resulting solution increases in weight to an extent sufficient to correspond to the desired sulphite or bisulphite salt. For example, to prepare a solution which is substantially a bisulphite salt, twice as much weight increase is needed as for the corresponding sulphite salt. If the amine or quaternary ammonium compound is not fully soluble (or fully in a dissolved form) at the start of the sulphur dioxide gas addition (but is partially dispersed or suspended in the aqueous phase), it becomes completely dissolved as the $SO_2$ addition continues. The solutions made from the various corresponding amines appear to exist most conveniently in solution form, although some if desired may be obtained as solids, e.g., monoethyl ammonium sulphite. Solutions may be diluted to 1 to 10 percent and are preferably stored in closed containers to reduce oxidation. An alternative synthesis procedure is to use an ion exchange resin in the sulphite form, if desired. Some of the solutions made from various corresponding quaternary compounds, after preparation, can subsequently be evaporated to dry solid form, such as tetramethyl ammonium bisulphite; others appear to exist only in solution form.

Salts of carbonyl sulphur dioxide adducts can be prepared by any convenient techniques. One currently preferred starting material for making a compound of the formula in (C) is formaldehyde.

Sodium formaldehyde bisulphite may be purchased commercially.

One convenient technique of preparation is to agitate a saturated aqueous solution of the desired bisulphite salt with less than a stoichiometric amount of a carbonyl compound which corresponds to the carbonyl sulphur dioxide adduct desired. Solutions containing more than about 60 weight percent of water are preferred. Other known synthesis methods may be used, if desired.

Since aqueous solutions of sulphur dioxide display a capacity to lengthen PT and PTT values at least to the extent indicated above, aqueous solutions of sulphur dioxide could be used in combination with, e.g. in admixture with, aqueous solutions of compounds of the formulae in (A), (B) and (C) above identified.

A preferred process for treating a thrombotic condition in a mammal (including man), with a composition of the present invention, includes the following steps:

(A) determining from a sample of blood removed from the mammal

(1) one or both of the blood coagulation factors consisting of prothrombin time and partial thromboplastin time and

(2) the platelet aggregation factor,

(B) when such factors fall within normal ranges for such mammal, administering to such mammal a pharmaceutical composition according to the invention at a dosage rate and for a time sufficient to inhibit the blood coagulation and platelet aggregation factors to an extent which is at least sufficient to inhibit thrombus development in the mammal but insufficient to cause spontaneous internal bleeding,

(C) at intervals during administration, removing further samples of blood from the mammal and determining the lengthened blood coagulation factor(s) and the inhibited platelet aggregation factor,

(D) adjusting administration of the composition so as to achieve and maintain predetermined desired values for the lengthened blood coagulation factor(s) and the inhibited platelet aggregation factor in the mammal, and

(E) continuing steps (B), (C) and (D) until the mammal has recovered from the thrombotic condition.

The compositions of the present invention can also be used *in vivo* or *in vitro* for inhibiting blood coagulation factors and for inhibiting platelet aggregation involving adding to blood and/or plasma derived from such blood an active agent of this invention.

Unless otherwise indicated herein, plasma is prepared by centrifuging whole blood at 2,000 r.p.m. Also, unless otherwise indicated, the term "control" in relation to plasma indicates a prothrombin time (PT) or a partial thromboplastin time (PTT) as determined with normal saline solution (equal volume).

The present invention is further illustrated by the following Examples. Reference may also be made to the disclosures, including specific Examples, contained in copending European Application Nos. 82200090.7 (EP—A—55996) and 82200091.5 (EP—A—55702) relating respectively to inorganic compounds and monosaccharide complex compounds and divided out of the present

disclosure. It is considered that a fuller understanding of the respective inventions can be obtained from the various disclosures when taken in conjunction.

Example A

A 10 weight percent aqueous solution of triethylamine-bisulphite was prepared by bubbling $SO_2$ through an appropriate solution or dispersion of triethylamine in water.

Example B

Using the procedure of Example A, a 10 weight percent aqueous solution of diethylaminebisulphite was prepared from diethylamine.

Example C

Using the procedure of Example A, a 10 weight percent aqueous solution of isobutylamino-bisulphite was prepared from isobutylamine.

Example D

Using the procedure of Example A, a 5 weight percent aqueous solution of *n*-octylamine-bisulphite was prepared from *n*-octylamine.

Example E

Using the procedure of Example A, a 5 weight percent solution of tributylamine-bisulphite was prepared from tributylamine.

Example F

Using the procedure of Example A, a 5 weight percent solution of ethylene-diamine-bisulphite was prepared from ethylene-diamine.

Example G

Using the procedure of Example A, a 5 weight percent solution of morpholine-bisulphite was prepared from morpholine.

Example H

Using the procedure of Example A, a 5 weight percent solution of cyclohexylamine-bisulphite was prepared from cyclohexylamine.

Example I

Using the procedure of Eample A, a 10 weight percent solution of triethylamine-sulphite was prepared from triethylamine. Half as much $SO_2$ was consumed as in the preparation of Example *A*.

Example J

A 10 weight percent aqueous solution of tetramethyl-ammonium-bisulphite was prepared by bubbling $SO_2$ through a solution of tetramethyl-ammonium hydroxide.

Example K

Using the procedure of Example J, a 10 weight percent aqueous solution of tetraethyl-ammonium-bisulphite was prepared from tetraethyl-ammonium hydroxide.

Example L

Using the procedure of Example J, a 10 weight percent aqueous solution of trimethyl-ethyl-ammonium-bisulphite was prepared from trimethyl-ethyl-ammonium hyroxide.

Example M

Using the procedure of Example J, a 5 weight percent solution of tetrabutyl-ammonium bisulphite was prepared from tetrabutyl-ammonium hydroxide.

Example N

A sample of the tetramethyl-ammonium bisulphite of Example J was evaporated under vacuum to dryness in a Buchi rotary evaporator. When all the water had been removed, an oil bath heated to 130°C was applied to the flask containing the solid and heating under vacuum was continued for another 4 hours, so as to remove water from the bisulphite and produce the metabisulphite.

Example O

A 5 weight percent aqueous solution of tetramethyl-ammonium sulphite was prepared by bubbling $SO_2$ through a solution of tetramethyl-ammonium hydroxide.

Example P

A solution of sodium formaldehyde bisulphite was prepared by dissolving commercially-available solid sodium formaldehyde bisulphite in distilled water at room temperature to form a 3 percent by weight aqueous solution.

Example Q

Example P was repeated except that a 10 percent by weight aqueous solution was formed.

Example R

Capsules of sodium formaldehyde bisulphite were prepared by charging standard gelatin capsules with sufficient sodium formaldehyde bisulphite to provide 25 mg of active agent per capsule.

Example S

The procedure of Example R was repeated, except that capsules containing 50 mg of active agent were prepared.

Example 1

A variety of agents were coded and evaluated under a code designation. Among these compounds, thiouracil, mercaptosuccinic acid, thiosemicarbazide, phenothiazine and sodium thiosulphate were included, so as to compare sodium bisulphite with certain compounds mentioned in USP 4,148,855.

After determination of PT and PTT values, the coded compounds were decoded. The bisulphites and sulphites showed an effect *in vitro* and only one other compound (sodium formaldehyde bisulphite) was effective in increasing PT and PTT values.

The results are shown in Table I where mean values of 5 replications in each case are reported. The findings demonstrate the reliability of the test method. This procedure was repeated with some additional compounds on two different days using a different control on each day. The results are shown in Table II below. These data indicate that the active moieties are probably the bisulphite and sulphite groups.

TABLE I
Effects of coded agents (0.5 mg/ml)

| L.C. LAB | Unknown reagents (code designation) | No. (replications) | PT (sec) | No. (replications) | PTT (sec) | Agent identification (decoded) |
|---|---|---|---|---|---|---|
| | Control | 5 | 12.92 | 5 | 43.38 | |
| | $A_2$ | 5 | 14.60 | 5 | 48.70 | Mercaptosuccinic acid |
| | $A_3$ | 4 | 14.95 | 3 | 53.00 | Dihydroxymalic acid $2H_2O$ |
| | $SO_2$ | 5 | 14.94 | 5 | 49.22 | Sodium formaldehyde sulphoxalate |
| | $SO_3$ | 5 | 19.50 | 5 | 58.60 | Sodium formaldehyde bisulphite |
| | $SO_4$ | 5 | 14.02 | 5 | 49.60 | Sodium diethyl dithiocarbamate |
| | $SI_1$ | 5 | 19.34 | 5 | 83.84 | Potassium metabisulphite |
| | $SI_2$ | 5 | 16.14 | 5 | 64.62 | Sodium sulphite |
| | $SI_4$ | 5 | 23.06 | 5 | 114.80 | Sodium bisulphite |
| | Control | | 13.0 | | 44.7 | |
| | $A_4$ | | 12.70 | | 51.8 | L-Ascorbic acid |
| | $N_1$ | | 13.1 | | 47.5 | D-(—)Ascorbic acid |
| | $N_2$ | | 12.8 | | 45.4 | Thiouracil |
| | $N_3$ | | 12.7 | | 43.9 | Carbohydrazide |
| | $N_4$ | | 13.0 | | 47.6 | Thiosemicarbazide |
| | $SO_1$ | | 12.9 | | 45.4 | Sodium p-toluene sulphinate |
| | $SI_3$ | | 13.6 | | 43.0 | Sodium phosphate |
| | $SI_5$ | | 13.4 | | 44.2 | Sodium hypophosphite |
| | $SI_6$ | | 13.5 | | 42.4 | Sodium thiosulphite |

TABLE II
Effects of various agents (0.5 mg/ml)

| Reagents | No. Exp. (replications) | PT (sec) | No. Exp. (replications) | PTT (sec) |
|---|---|---|---|---|
| Control | 5 | 13.40 | 4 | 42.6 |
| $Na_2S_2O_5$ | 5 | 19.56 | 4 | 77.75 |
| $NaHSO_3$ | 5 | 22.42 | 4 | 120.35 |
| Na sulphide | 5 | 21.12 | 4 | 78.50 |
| $Na_2SO_3$ | 5 | 16.38 | 4 | 66.53 |
| $(NH_4)_2SO_3$ | 5 | 16.08 | 4 | 58.80 |
| Na-formaldehyde—$HSO_3$ | 5 | 19.68 | 3 | 56.30 |
| $K_2H_2SO_5$ | 5 | 18.76 | 4 | 80.60 |
| Control | — | 13.40 | — | 42.10 |
| Na-phosphite | 1 | 12.9 | — | — |
| Na-hydrosulphite | 2 | 14.4 | — | 45.2 (39.0) |
| Mercaptosuccinic acid | 1 | 14.7 | 1 | 52.6 |
| Na-thiosulphate | 1 | 13.5 | 1 | 46.2 |
| $Na_2SO_4$ | 1 | 13.6 | 1 | 44.2 |
| Na-diethylthiocarbonate | 1 | 13.6 | 1 | 48.1 |
| Na-Hypophosphite | 1 | 13.9 | 1 | 47.19 (53.8) |
| Na-formaldehyde sulphoxalate | 1 | 13.8 | 1 | 45.1 (53.8) |

Example 2

Solution A was found to prolong PT and PTT in a dose-related fashion. When added to human and to rabbit plasma *in vitro*, the agent triethyl-ammonium bisulphite was found to prolong PT and PTT significantly at a concentration of 0.5 mg/ml and the effects are directly proportional to dose. The agent is active in inhibiting various coagulation factors, including factors VII, IX, X, XI, and XII.

References for PT, PTT and assays of all the coagulation factors can be found in the above-cited textbook, "Human Blood Coagulation, Haemostasis and Thrombosis".

Examples 3 and 4

When solution A and Solution I were each further similarly tested as in Example 2, increases in PT and PTT values at similar respective concentrations are observed, although somewhat different values are obtained.

Data evaluated by the procedure above described are provided in Tables III and IV below.

Example 5

To demonstrate that triethylamine sulphite and triethylamine bisulphite have an effect upon blood coagulation factors, the data in Table III (below) for fibrinogen, Factor IX and Factor X using Biggs procedure were obtained. The results show a substantial effect on these factors, with the bisulphite being the more active.

Example 6

To demonstrate reproducibility of the Biggs procedure, the work of Example 5 was repeated 5 times and the deviation is summarized and shown in Table IV below.

Example 7

A rabbit was injected with about 72 mg/kg of body weight of triethylamine bisulphite. After a

period of 5 hours, a blood sample (220 g) was withdrawn and centrifuged at 1000 r.p.m. for 10 minutes to give a platelet-rich plasma. The material was evaluated for platelet aggregation by the method described by Born (cited above) and it was found that aggregation of platelets was markedly decreased in comparison with an untreated rabbit. It is concluded that triethylamine bisulphite is an agent which inhibits thrombotic condition formation *in vivo.*

Other amine bisulphites, such as *n*-octylamine bisulphite and tri-*n*-butylamine bisulphite, also demonstrate similar inhibition of platelet aggregation when similarly tested.

Example 8

Platelet-rich plasma from rabbits and men was prepared by the centrifugation technique mentioned in Example 7. Using ADP (adenosine diphosphate) as a stimulus, rapid aggregation of platelets occurred. When the stimulated blood had been treated with each of the agents of Example 7, there was a strong inhibition of platelet aggregation. This procedure is described in the article by Born in Nature, Vol. 194, pgs. 927—929 (1962). The results demonstrate that these agents cause inhibition of platelet aggregation *in vitro.*

Other stimuli, collagen and arachidonic acid, yielded similar results.

TABLE III

Effect of amine derivatives on selected coag. factor

Factor: fibrinogen, factors IX and X

| Exp. No. | Agent resignation | Fibrinogen (%)[1] | Factor IX (%)[2] | Factor X (%)[3] |
|---|---|---|---|---|
| 4.1 | Control | 205 | 50 | 100 |
| 4.2 | Triethyl amine bisulphite | 125 | 14 | 14 |
| 4.3 | Triethyl amine sulphite | 170 | 19 | 23 |

Table III Footnotes:

[1]Values shown represent mean of two experiments.

[2]Value of a single experiment.

[3]Mean of three experiments.

Table IV
Effect of amine derivatives on coagulation

| Example No. | Experiment No. No. 1 PT (sec) | No. 1 PTT (sec) | No. 2 PT (sec) | No. 2 PTT (sec) | No. 3 PT (sec) | No. 3 PTT (sec) | No. 4 PT (sec) | No. 4 PTT (sec) | No. 5 PT (sec) | No. 5 PTT (sec) | Mean and LSD (least standard deviation) PT (sec) | Mean and LSD (least standard deviation) PTT (sec) |
|---|---|---|---|---|---|---|---|---|---|---|---|---|
| 5.1 Control | 12.9 | 36.7 | 12.9 | 36.2 | 11.4 | 36.4 | 12.0 | 36.0 | 12.1 | 38.9 | 12.3 ±0.6 | 36.8 ±1.2 |
| 5.2 Triethylamine bisulphite | 23.3 | 24.7 | 25.3 | 86.3 | 18.5 | 64.2 | 18.9 | 71.2 | 20.4 | 68.9 | 21.3 ±3.0 | 73.1 ±8.3 |
| 5.3 Triethylamine sulphite | 17.0 | 59.2 | 18.5 | 65.1 | 15.4 | 54.9 | 16.6 | 59.0 | 16.8 | 64.0 | 16.9 ±1.1 | 60.6 ±4.1 |

PT=Prothrombin time (which measures integrity of the extrinsic coag. pathway).
PTT=Partial prothrombin time (which measures integrity of the intrinsic pathway).

Example 9

The procedure of Biggs was repeated for PT and PTT determination using various compounds of the formula in (A), with multiple replications for accuracy and consistency reasons. These compounds are generally effective in increasing PT and PTT. In general, the bisulphites of the Formula in (A) are somewhat more effective in increasing PT and PTT values than are the sulphites; the bisulphites of diamines (as compared with monoamines) are effective. Such an increase indicates an anticoagulation effectiveness *in vitro*. The diallyl-amine bisulphite is unexpectedly of low activity in increasing PT and PTT values.

The results are shown in Table V below.

Even though aromatic amines, such as aniline, form active agents, they are considered to be excessively toxic for mammalian use and so are not included within the scope of the formula in (A).

The alkanolamine bisulphites are of approximately the same activity as the lower alkyl amine bisulphites.

Example 10

To evaluate whether or not an amine moiety has an effect on blood factors, some amines were evaluated as their hydrochlorides (as opposed to the corresponding sulphites and bisulphites).

After their respective preparations the amine salts were evaluated by the Biggs method for their effect on PT and PTT. The data shown in Table VI below show that these hydrochlorides have no effect upon PT and PTT *in vitro*.

TABLE V

Effect of amine bisulphites on blood coagulation

| | PTT Replications (sec) | | | PT Replications (sec) | | |
|---|---|---|---|---|---|---|
| | 1 | 2 | 3 | 1 | 2 | 3 |
| 7.1 N-Methyl Cyclohexyl Amine | 60.6 | 61.8 | 61.6 | 20.5 | 20.3 | 20.4 |
| 7.2 Allyl Amine | 47.1 | 46.9 | 44.9 | 16.1 | 16.0 | 15.6 |
| 7.3 Tert-Butyl Amine | 68.2 | 68.9 | 69.0 | 21.7 | 21.8 | 21.8 |
| 7.4 Diallyl Amine | 30.8 | 39.8 | 39.1 | 13.4 | 13.7 | 13.8 |
| 7.5 Pyridine | 80.0 | 71.1 | 59.8 | 29.3 | 27.8 | 27.2 |
| 7.6 N,N-Dimethyl Cyclohexyl Amine | 60.1 | 62.1 | 58.9 | 18.9 | 20.4 | 19.8 |
| 7.7 Morpholine | 66.8 | 61.8 | 51.7 | 19.3 | 21.0 | 17.5 |
| 7.8 Aniline | 45.4 | 44.9 | 42.9 | 16.0 | 16.9 | 16.0 |
| 7.9 Ethanol Amine | 65.3 | 66.1 | 61.1 | 19.3 | 20.5 | 18.4 |
| 7.10 Control Plasma | 37.5 | 39.5 | 37.8 | 12.8 | 12.8 | 13.1 |
| 7.11 4-Methyl Morpholine | 63.5 | 61.3 | 60.6 | 21.3 | 21.5 | 20.4 |
| 7.12 Diethanol Amine | 62.6 | 58.0 | 60.6 | 20.6 | 19.8 | 19.7 |
| 7.13 Triethanol Amine | 74.2 | 68.8 | 73.6 | 22.9 | 23.1 | 23.3 |
| 7.14 Control Plasma | 38.2 | 38.3 | 39.2 | 13.1 | 13.1 | 13.0 |

TABLE VI
Effect of amine hydrochlorides on blood coagulation

| | PT (sec) replications | | | PTT (sec) replications | | |
|---|---|---|---|---|---|---|
| | 1 | 2 | 3 | 1 | 2 | 3 |
| 9.1 Triethyl Amine Hydrochloride | 12.8 | 12.7 | 13.5 | 32.0 | 31.5 | 34.5 |
| 9.2 Cyclohexyl Amine Hydrochloride | 13.8 | 13.6 | 13.5 | 32.5 | 32.7 | 34.3 |
| 9.3 Allyl Amine Hydrochloride | 13.2 | 13.2 | 14.4 | 31.6 | 30.5 | 32.4 |
| 9.4 Diallyl Amine Hydrochloride | 12.8 | 13.1 | 13.5 | 30.5 | 30.7 | 30.4 |
| 9.5 Pyridine Hydrochloride (Crystals) | 12.9 | 12.8 | 14.0 | 31.3 | 31.1 | 32.2 |
| 9.6 Pyridine Hydrochloride (Powder) | 12.6 | 12.5 | 13.8 | 30.5 | 30.8 | 31.1 |
| 9.7 Control Plasma | 13.0 | 12.5 | 13.5 | 31.5 | 31.3 | 31.2 |

Example 11

A rabbit was continuously infused with aqueous triethylamine bisulphite solution at a rate of 0.001 kg per minute (5.514 gram per hour) for a time of five hours. After 5 hours, the animal was sacrificed and the radioactive fibrinogen accumulated on the damaged jugular vein was determined. The radioactivity in the contra-lateral (undamaged) jugular vein was also determined as a control. Concurrently, another rabbit was subjected to the same procedure, but received only sodium chloride solution as a control. It was found to have 7 times as much radioactivity as the counterpart infused with triethylamine bisulphite. Thus, triethylamine bisulphite has a definite positive antithrombotic effect in the jugular vein.

When *n*-octylamine bisulphite, tributylamine bisulphite, diethanolamine bisulphite and triethylamine sulphite were similarly evaluated, it was concluded that such compounds also showed a definite positive antithrombotic effect in the jugular vein.

Example 12

Solutions C and D both cause an increase in PT and PTT when added to plasma at 0.5 mg/ml.

Example 13

Triethylamine bisulphite was evaluated under *in vivo* conditions using an anti-indium$^{111}$ platelet labelling technique in rabbits (see Wu et al above cited). The material was administered by intravenous injection (127.39 mg/kg of body weight), equivalent to 0.01 molar concentrations in blood.

The active agent effected a reduction of about 30% in the accumulation of platelet thrombus. Another rabbit so tested died.

Example 14

Solution K was found to prolong PT and PTT in a dose-related fashion. When added to human or rabbit plasma *in vitro*, the agent tetramethylammonium bisulphite was found to prolong PT and PTT significantly at a concentration of 0.5 mg/ml and the effects are proportional and therefore dose-related. The agent is active in inhibiting various coagulation factors, including VII, IX, X, XI, and XII.

Example 15

The procedure of Example 14 was repeated using solution L and similar results were obtained.

Example 16

The procedure of Example 14 was repeated using solution M and similar results were obtained.

Example 17

Solutions J and O were used according to the procedure of Biggs to determine their effect upon PT and PTT. Data from five replications are shown in Table VII below. (The concentration of active agent employed was 0.5 mg/ml in each instance). Both solutions are effective for lengthening blood coagulation time but the tetraethylammonium bisulphite is more active.

Example 18.

Solutions J and O were used according to the procedure of Biggs to evaluate their effect upon

fibrinogen and Factors IX and X. The data shown in Table VIII indicate a strong effect upon Factors IX and X and a relatively mild effect upon fibrinogen (Factor I).

Example 19

Plasma (200 g) was centrifuged at 1000 r.p.m. to prepare a platelet-rich plasma. Using adenosine diphosphate (ADP) as a stimulus, rapid aggregation of platelets in the plasma was effected. On the other hand, when such plasma is first treated with (a) tetramethylammonium sulphite, (b) tetramethylammonium bisulphite and then (c) tetrabutylammonium bisulphite, it is found that platelet aggregation in each instance is inhibited, indicating that each of these compounds is a potent inhibitor of platelet aggregation.

TABLE VII
Effect of quaternary amine derivatives on coagulation

Experiment no.

| | #1 | | #2 | | #3 | | #4 | | #5 | | Mean LSD | |
|---|---|---|---|---|---|---|---|---|---|---|---|---|
| | PT (1) (sec) | PTT (sec) | PT (sec) | PTT (sec) | PT (sec) | PTT (sec) | PT (sec) | PTT (sec) | PT (sec) | PTT (sec) | PT (sec) | PTT (sec) |
| Control | 12.9 | 36.7 | 12.9 | 36.2 | 14.4 | 36.4 | 12.2 | 36.0 | 12.1 | 38.9 | 12.3± 0.6 | 38.8± 1.2 |
| Tetramethyl-ammonium bisulphite | 21.7 | 73.4 | 23.7 | 86.7 | 17.8 | 66.2 | 19.1 | 70.0 | 20.1 | 77.0 | 20.5± 2.3 | 74.7± 7.8 |
| Tetramethyl-ammonium sulphite | 17.5 | 59.0 | 19.2 | 67.3 | 16.1 | 57.9 | 17.4 | 61.8 | 18.2 | 70.0 | 17.7± 1.1 | 63.2± 5.3 |

(1) PT=Prothrombin time (which measures the integrity of extrinsic coagulation pathway)
PTT=Partial thromboplastin time (which measures the integrity of intrinsic pathway)

TABLE VIII
Effect of quaternary amine derivatives on selected coagulation

Factors: Fibrinogen, Factor IX and Factor X

| | Fibrinogen (%)[1] | Factor IX (%)[2] | Factor X (%)[3] |
|---|---|---|---|
| Control | 205 | 50 | 100 |
| Tetramethyl-ammonium bisulphite | 170 | 12.5 | 10 |
| Tetramethyl-ammonium sulphite | 175 | 20 | 25 |

[1] Values shown represent mean of 2 experiments
[2] Value of a single experiment
[3] Mean of 3 experiments

Example 20

To demonstrate that it is not the quaternary ammonium group which is affecting blood factors, two tests were run evaluating the effect of tetramethyl ammonium bromide and tetrabutyl ammonium bromide (Biggs) *in vitro* on PT and PTT using human plasma. The data shown in Table IX indicate that these compounds have little or no effect on PT and PTT when compared to the effect produced by the corresponding bisulphites.

Example 21

A rabbit was continuously infused with aqueous tetrabutyl-ammonium bisulphite solution at the rate of .001 mg/kg/min (5.154 gram per hour) for a time of five hours. After 5 hours, the procedure of Example 11 was followed and showed that tetrabutyl-ammonium bisulphite shows a definite positive effect of decreasing thrombus formation in the jugular vein.

The same procedure using tetramethylammonium bisulphite produced death of the rabbit.

Example 22

Evaluation of the antithrombotic effect of sodium formaldehyde bisulphite was carried out on a rabbit as described in Kelton et al: "Thrombogenic Effect of High Dose Aspirin® in Rabbits", Journal of Clinical Investigation, Volume 62, pages 892—895, 1978. When the rabbit was treated with 50 mg/kg of sodium formaldehyde bisulphite $CH_2SO_3Na(OH)$, the thrombus disappeared more rapidly than in a control animal.

TABLE IX
Effect of tetra alkyl ammonium bromides
0.5 mg/ml

| | PT(sec) | PTT(sec) |
|---|---|---|
| Tetramethyl Ammonium Bromide | 12.5 | 38.5 |
| Tetrabutyl Ammonium Bromide | 12.4 | 38.3 |
| Control | 12.3 | 38.1 |
| Tetramethyl Ammonium Bisulphite | 21.7 | |
| Control | 12.9 | |

Example 23

A rabbit was fed orally by gavage about 50 mg/kg sodium formaldehyde bisulphite in two spaced doses of the solution of Example Q. After a period of 1 day, the animal's blood was analyzed for PT and PTT values and it was found that both values had increased as compared with corresponding values from the blood of an otherwise untreated control rabbit.

Example 24

Using the procedure described in the Biggs reference, PT and PTT values for sodium formaldehyde bisulphite were obtained as shown in Table X below:

TABLE X

| | PT | PTT |
|---|---|---|
| Control | 12.5 | 33.4 |
| 0.01 Molar Sodium formaldehyde bisulphite | 24.4 | 54.6 |

Example 25

Two jugular veins on a normal healthy rabbit were exposed and one of the veins was systematically damaged by clamping in two longitudinally-spaced places. The rabbit was then continuously infused with aqueous sodium formaldehyde bisulphite at the rate of .001 kg per minute (5.154 gram per hour) (animal body weight 2.3 kg). The rabbit was then injected with fibrinogen labelled with $I^{125}$. After 5 hours, the damaged jugular vein was evaluated for radioactivity with a Geiger counter. When the amount of radioactivity in the rabbit infused with sodium formaldehyde bisulphite was compared to the count of the jugular vein of a rabbit infused with normal saline solution, the latter rabbit had more radioactivity than the former.

It is concluded that sodium formaldehyde bisulphite shows a definite positive effect of decreasing thrombus formation in the jugular vein. Closely similar results were achieved with a second rabbit. Other rabbits, similarly evaluated, died, apparently because of overdose by the infusion technique.

Example 26

A rabbit was injected with about 72 mg/kg of body weight of sodium formaldehyde bisulphite. After a period of 5 hours, a blood sample was withdrawn and centrifuged at 1000 r.p.m. (220 g) for 10 minutes to give a platelet-rich plasma. The material was evaluated for platelet aggregation by the method described in Wu et al (cited above) and it was found that aggregation of platelets was markedly decreased in comparison with an untreated rabbit. It is concluded that sodium formaldehyde bisulphite is an agent which inhibits thrombus formation *in vivo*. Similar test results were achieved with other rabbits so tested. It is estimated that this material is 1000 times less effective than sodium bisulphite.

Example 27

Platelet-rich plasma from rabbits and men was prepared by the centrifugation technique referenced in Example 7. Using ADP (adenosine diphosphate) or arachidonic acid as a stimulus, rapid aggregation of platelets occurred. When the stimulated blood had been treated with sodium formaldehyde bisulphite, there was a strong inhibition of platelet aggregation. The results demonstrate that sodium formaldehyde bisulphite causes inhibition of platelet aggregation *in vitro*.

Example 28

Bisulphite adducts were prepared from acetone, cyclohexanone, benzaldehyde and 2-octanone. When evaluated by the Biggs technique, they had a minimal effect on PT and PTT at 0.5 mg/ml.

As is apparent from the foregoing specification, the present invention can be carried out with a wide variety of alterations and modifications. For this reason, it is to be fully understood that all of the foregoing is intended to be merely illustrative and is not to be taken as restrictive or otherwise limiting of the present invention, excepting as defined in the appended claims.

**Claims for the Contracting States: BE CH DE FR GB IT LI LU NL SE**

1. A pharmaceutical composition for use in treating an actual or incipient thrombotic condition in a mammal, characterized in that it contains at least one carrier substance and, as the active treating agent, at least one compound selected from:

(A) compounds of the formula:

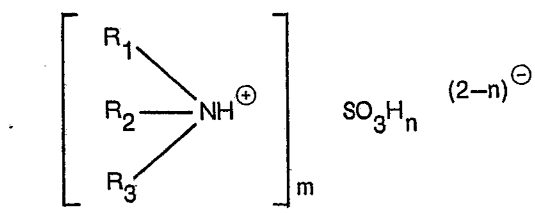

wherein:

$R_1$ is an alkyl radical containing less than 11 carbon atoms, a cyloalkyl radical containing 6 to 10 carbon atoms or a monohydroxyalkyl radical containing less than 11 carbon atoms,

$R_2$ is a hydrogen atom, an alkyl radical containing less than 11 carbon atoms or a monohydroxyalkyl radical containing less than 11 carbon atoms, or

$R_1$ and $R_2$ together form an unsubstituted morpholine, piperidine or hexamethyleneimine ring or such a ring carrying on one of its carbon atoms an alkyl radical containing less than 11 carbon atoms,

$R_3$ is a hydrogen atom, an alkyl radical containing less than 11 carbon atoms, a monohydroxyalkyl radical containing less than 11 carbon atoms or a radical of the formula:

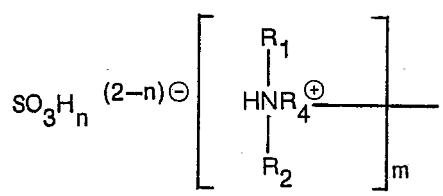

wherein:

$R_1$ and $R_2$ have the meanings defined above,

$R_4$ is a divalent saturated aliphatic radical containing less than 11 carbon atoms, and

m is 1 or 2, n is 0 or 1 and the sum of m and n is 2;

(B) compounds of either of the formulae:

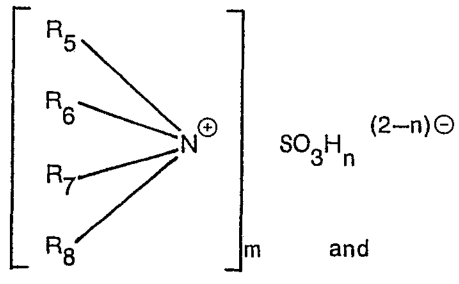

and

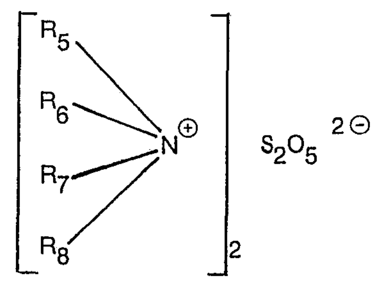

wherein:

$R_5$, $R_6$, $R_7$ and $R_8$ are the same or different and each is an alkyl radical, containing less than 11 carbon atoms, and

m and n have the meanings defined above;

(C) compounds of the formula:

$$R—CHOH—SO_3M$$

wherein:

R is a hydrogen atom or a hydroxymethyl radical and

M is an alkali metal or an ammonium group, but excluding:

(a) 0.6M injectable aqueous solutions of ethanolamine bisulphite, and

(b) compositions containing a formaldehyde bisulphite and a protamine.

2. A pharmaceutical composition as claimed in Claim 1, wherein the above treating agent is a compound of type (A) in which $R_1$, $R_2$ and $R_3$ each represent an alkyl group containing 1 to 4 carbon atoms.

3. A pharmaceutical composition as claimed in Claim 2, wherein $R_1$, $R_2$ and $R_3$ each represent ethyl.

4. A pharmaceutical composition as claimed in Claim 1, wherein the active treating agent is a compound of type (A) in which $R_1$ represents an alkyl group containing 5 to 10 carbon atoms and $R_2$ and $R_3$ both represent hydrogen.

5. A pharmaceutical composition as claimed in Claim 4, wherein $R_1$ represents *n*-octyl.

6. A pharmaceutical composition as claimed in Claim 1 wherein the active treating agent is a compound of type (B) in which $R_5$, $R_6$, $R_7$ and $R_8$ each represent an alkyl group containing 1 to 4 carbon atoms.

7. A pharmaceutical composition as claimed in Claim 6, wherein $R_5$, $R_6$, $R_7$ and $R_8$ each represent butyl.

8. A pharmaceutical composition as claimed in Claim 1, wherein the active treating agent is sodium formaldehyde bisulphite.

9. A pharmaceutical composition as claimed in any one of the preceding Claims, wherein the active treating agent is a bisulphite.

10. A pharmaceutical composition as claimed in any one of the preceding Claims, wherein the composition is in an orally administrable form.

11. A pharmaceutical composition as claimed in Claim 10, wherein the composition is an aqueous solution containing 1 to 10% by weight of the active treating agent.

12. A pharmaceutical composition as claimed in any one of Claims 1 to 9, wherein the composition is in an injectable form.

13. A pharmaceutical composition as claimed in Claim 12, wherein the composition is an injectable aqueous solution containing 1 to 11% by weight of the active treating agent.

14. A pharmaceutical composition as claimed in any one of the preceding Claims, wherein the active treating agent is the only pharmacologically active agent in the composition.

15. A pharmaceutical composition as claimed in any one of Claims 1 to 13 wherein the composition is in the form of an aqueous solution containing dissolved sulphur dioxide.

16. A compound as defined in any one of Claims 1 to 9 for use in treating an actual or incipient thrombotic condition in a human or other mammal.

**Claims for the Contracting State: AT**

1. A method of preparing a pharmaceutical composition for use in treating an actual or incipient thrombotic condition in a mammal, characterized in that it comprises mixing at least one carrier substance with, as the active treating agent, at least one compound selected from:

(A) compounds of the formula:

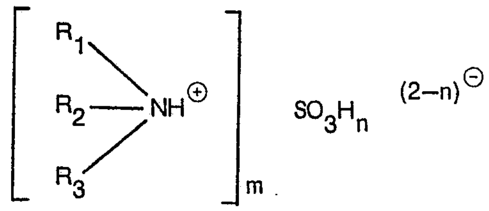

wherein:

$R_1$ is an alkyl radical containing less than 11 carbon atoms, a cycloalkyl radical containing 6 to 10 carbon atoms or a monohydroxyalkyl radical containing less than 11 carbon atoms,

$R_2$ is a hydrogen atom, an alkyl radical containing less than 11 carbon atoms or a monohydroxyalkyl radical containing less than 11 carbon atoms, or

$R_1$ and $R_2$ together form an unsubstituted morpholine, piperidine or hexamethyleneimine ring or such a ring carrying on one of its carbon atoms an alkyl radical containing less than 11 carbon atoms,

$R_3$ is a hydrogen atom, an alkyl radical containing less than 11 carbon atoms, a monohydroxyalkyl radical containing less than 11 carbon atoms or a radical of the formula:

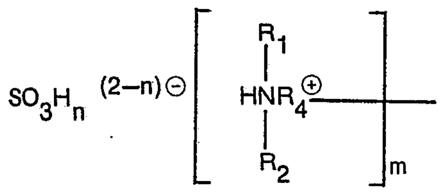

wherein:

$R_1$ and $R_2$ have the meanings defined above,

$R_4$ is a divalent saturated aliphatic radical containing less than 11 carbon atoms, and

m is 1 or 2, n is 0 or 1 and the sum of m and n is 2;

(B) compounds of either of the formulae:

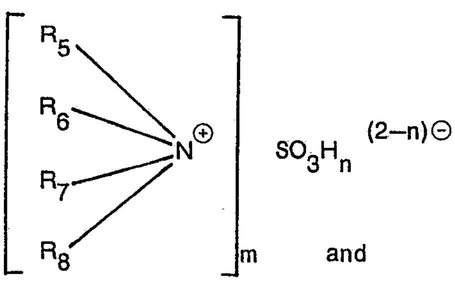

and

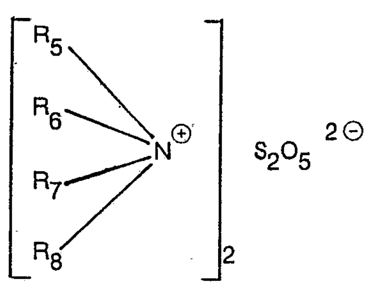

wherein:

$R_5$, $R_6$, $R_7$ and $R_8$ are the same or different and each is an alkyl radical, containing less than 11 carbon atoms, and

m and n have the meanings defined above;

(C) compounds of the formula:

$$R—CHOH—SO_3M$$

wherein:

R is a hydrogen atom or a hydroxymethyl radical and

M is an alkali metal or an ammonium group, but excluding:

(a) 0.6M injectable aqueous solutions of ethanolamine bisulphite, and

(b) compositions containing a formaldehyde bisulphite and a protamine.

2. A method as claimed in Claim 1, wherein the above treating agent is a compound of type (A) in which $R_1$, $R_2$ and $R_3$ each represent an alkyl group containing 1 to 4 carbon atoms.

3. A method as claimed in Claim 2, wherein $R_1$, $R_2$ and $R_3$ each represent ethyl.

4. A method as claimed in Claim 1, wherein the active treating agent is a compound of type (A) in which $R_1$ represents an alkyl group containing 5 to 10 carbon atoms and $R_2$ and $R_3$ both represent hydrogen.

5. A method as claimed in Claim 4, wherein $R_1$ represents *n*-octyl.

6. A method as claimed in Claim 1 wherein the active treating agent is a compound of type (B) in which $R_5$, $R_6$, $R_7$ and $R_8$ each represent an alkyl group containing 1 to 4 carbon atoms.

7. A method as claimed in Claim 6, wherein $R_5$, $R_6$, $R_7$ and $R_8$ each represent butyl.

8. A method as claimed in Claim 1, wherein the active treating agent is sodium formaldehyde bisulphite.

9. A method as claimed in any one of the preceding claims, wherein the active treating agent is a bisulphite.

10. A method as claimed in any one of the preceding claims, wherein the composition is in an orally administrable form.

11. A method as claimed in Claim 10, wherein the composition is an aqueous solution containing 1 to 10% by weight of the active treating agent.

12. A method as claimed in any one of Claims 1 to 9, wherein the composition as in an injectable form.

13. A method as claimed in Claim 12, wherein the composition is an injectable aqueous solution containing 1 to 11% by weight of the active treating agent.

14. A method as claimed in any one of the preceding claims, wherein the active treating agent is the only pharmacologically active agent in the composition.

15. A method as claimed in any one of Claims 1 to 13 wherein the composition is in the form of an aqueous solution containing dissolved sulphur dioxide.

**Patentansprüche für die Vertragsstaaten: BE CH DE FR GB IT LI LU NL SE**

1. Pharmazeutische Zusammensetzung zur Verwendung zur Behandlung einer akuten oder beginnenden Thrombose bei Säugern, dadurch gekennzeichnet, daß sie mindestens eine Trägersubstanz und als aktives Behandlungsmittel mindestens eine organische Verbindung enthält, die ausgewählt ist aus:

A) Verbindungen mit der Formel:

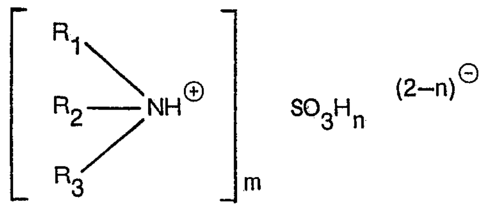

in der:

$R_1$ ein Alkylrest mit weniger als 11 Kohlenstoffatomen, ein Cycloalkylrest mit 6 bis 10 Kohlenstoffatomen oder ein Monohydroxyalkylrest mit weniger als 11 Kohlenstoffatomen ist,

$R_2$ ein Wasserstoffatom, ein Alkylrest mit weniger als 11 Kohlenstoffatomen oder ein Monohydroxyalkylrest mit weniger als 11 Kohlenstoffatomen ist oder

$R_1$ und $R_2$ zusammen einen nichtsubstituierten Morpholin-, Piperidin- oder Hexamethyleniminring oder einen derartigen Ring bilden, der an einem seiner Kohlenstoffatome einen Alkylrest mit weniger als 11 Kohlenstoffatomen aufweist,

$R_3$ ein Wasserstoffatom, ein Alkylrest mit weniger als 11 Kohlenstoffatomen, ein Monohydroxyalkylrest mit weniger als 11 Kohlenstoffatomen oder ein Rest mit der Formel:

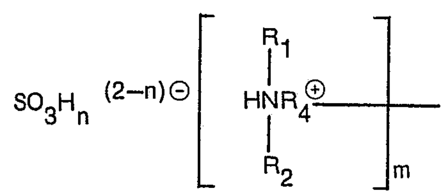

ist, in der $R_1$ und $R_2$ die oben angegebene Bedeutung besitzen,

$R_4$ ein zweiwertiger gesättigter aliphatischer Rest mit weniger als 11 Kohlenstoffatomen ist und m 1 oder 2, n 0 oder 1 und die Summe von m und n 2 ist;

B) Verbindungen mit einer der Formeln:

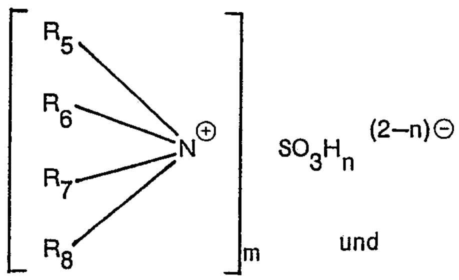

und

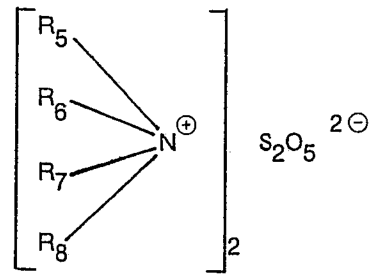

in denen $R_5$, $R_6$ $R_7$ und $R_8$ gleich oder verschieden und jeweils ein Alkylrest mit weniger als 11 Kohlenstoffatomen sind und
m and n die oben angegebene Bedeutung besitzen;

C) Verbindungen mit der Formel:

$$R—CHOH—SO_3M,$$

in der R ein Wasserstoffatom oder ein Hydroxymethylrest ist und M ein Alkalimetall oder eine Ammoniumgruppe ist, wobei aber ausgeschlossen sind:

a) 0,6 M injizierbare wässrige Lösungen von Ethanolaminbisulfit und
b) Zusammensetzungen, die ein Formaldehydbisulfit und ein Protamin enthalten.

2. Pharmazeutische Zusammensetzung nach Anspruch 1, dadurch gekennzeichnet, daß das obige Behandlungsmittel eine Verbindung vom Typ A) ist, in der $R_1$, $R_2$ und $R_3$ jeweils eine Alkylgruppe mit 1 bis 4 Kohlenstoffatomen bedeuten.

3. Pharmazeutische Zusammensetzung nach Anspruch 2, dadurch gekennzeichnet, daß $R_1$, $R_2$ und $R_3$ jeweils Ethylreste bedeuten.

4. Pharmazeutische Zusammensetzung nach Anspruch 1, dadurch gekennzeichnet, daß das aktive Behandlungsmittel eine Verbindung des Typs (A) ist, in der $R_1$ eine Alkylgruppe mit 5 bis 10 Kohlenstoffatomen und $R_2$ und $R_3$ beide Wasserstoff bedeuten.

5. Pharmazeutische Zusammensetzung nach Anspruch 4, dadurch gekennzeichnet, daß $R_1$ einen n-Octylrest bedeutet.

6. Pharmazeutische Zusammensetzung nach Anspruch 1, dadurch gekennzeichnet, daß das aktive Behandlungsmittel eine Verbindung des Typs B) ist, in der $R_5$, $R_6$, $R_7$ und $R_8$ jeweils eine Alkylgruppe mit 1 bis 4 Kohlenstoffatomen bedeuten.

7. Pharmazeutische Zusammensetzung nach Anspruch 6, dadurch gekennzeichnet, daß $R_5$, $R_6$, $R_7$ und $R_8$ jeweils einen Butylrest bedeuten.

8. Pharmazeutische Zusammensetzung nach Anspruch 1, dadurch gekennzeichnet, daß das aktive Behandlungsmittel Natriumformaldehydbisulfit ist.

9. Pharmazeutische Zusammensetzung nach einem der vorhergehenden Ansprüche, dadurch gekennzeichnet, daß das aktive Behandlungsmittel ein Bisulfit ist.

10. Pharmazeutische Zusammensetzung nach einem der vorangehenden Ansprüche, dadurch gekennzeichnet, daß die Zusammensetzung in oral verabreichbarer Form vorliegt.

11. Pharmazeutische Zusammensetzung nach Anspruch 10, dadurch gekennzeichnet, daß die Zusammensetzung eine wässrige Lösung ist, die 1 bis 10 Gew.% des aktiven Behandlungsmittels enthält.

12. Pharmazeutische Zusammensetzung nach einem der Ansprüche 1 bis 9, dadurch gekennzeichnet, daß die Zusammensetzung in injezierbarer Form vorliegt.

13. Pharmazeutische Zusammensetzung nach Anspruch 12, dadurch gekennzeichnet, daß die Zusammensetzung eine injzierbare wässrige Lösung ist, die 1 bis 11 Gew.% des aktiven Behandlungsmittels enthält.

14. Pharmazeutische Zusammensetzung nach einem der vorangehenden Ansprüche, dadurch gekennzeichnet, daß das aktive Behandlungsmittel der einzige pharmakologisch aktive Wirkstoff in der Zusammensetzung ist.

15. Pharmazeutische Zusammensetzung nach einem der Ansprüche 1 bis 13, dadurch gekennzeichnet, daß die Zusammensetzung in Form einer wässrigen Lösung vorliegt, die gelöstes Schwefeldioxid enthält.

16. Verbindung der Definition gemäß einem der Ansprüche 1 bis 9 zur Verwendung bei der Behandlung einer akuten oder beginnenden Thrombose bei Menschen oder anderen Säugern.

**Patentansprüche für den Vertragsstaat: AT**

1. Verfahren zur Herstellung einer pharmazeutischen Zusammensetzung zur Verwendung zur Behandlung einer akuten oder beginnenden Thrombose bei Säugern, dadurch gekennzeichnet, daß man mindestens eine Trägersubstanz mit mindestens einer organischen Verbindung als aktivem Behandlungsmittel vermischt welche ausgewählt ist aus:

A) Verbindungen mit der Formel

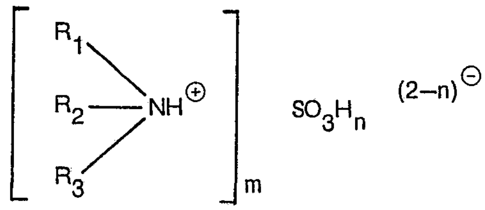

in der:

$R_1$ ein Alkylrest mit weniger als 11 Kohlenstoffatomen, ein Cycloalkylrest mit 6 bis 10 Kohlenstoffatomen oder ein Monohydroxyalkylrest mit wengier als 11 Kohlenstoffatomen ist,

$R_2$ ein Wasserstoffatom, ein Alkylrest mit weniger als 11 Kohlenstoffatomen oder ein Monohydroxyalkylrest mit weniger als 11 Kohlenstoffatomen ist oder

$R_1$ und $R_2$ zusammen einen nichtsubstituierten Morpholin-, Piperidin- oder Hexamethyleniminring oder einen derartigen Ring bilden, der an einem seiner Kohlenstoffatome einen Alkylrest mit weniger als 11 Kohlenstoffatomen aufweist,

$R_3$ ein Wasserstoffatom, ein Alkylrest mit weniger als 11 Kohlenstoffatomen, ein Monohydroxyalkylrest mit weniger als 11 Kohlenstoffatomen oder ein Rest mit der Formel:

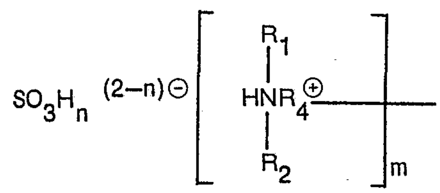

ist, in der $R_1$ und $R_2$ die oben angegebene Bedeutung besitzen,

$R_4$ ein zweiwertiger gesättigter aliphatischer Rest mit weniger als 11 Kohlenstoffatomen ist und

m 1 oder 2, n 0 oder 1 und die Summe von m und n 2 ist;

B) Verbindungen mit einer der Formeln:

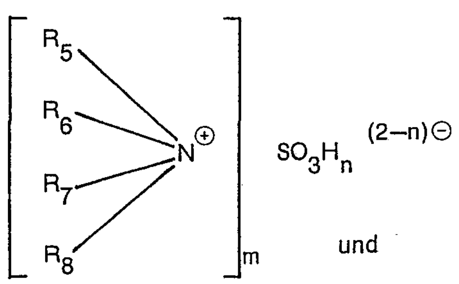

und

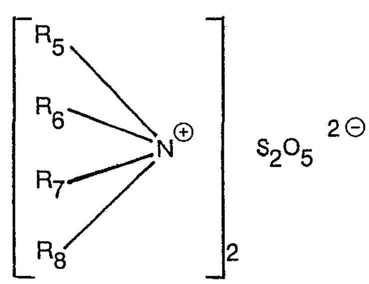

in denen $R_5$, $R_6$, $R_7$ und $R_8$ gleich oder verschieden und jeweils ein Alkylrest mit weniger als 11 Kohlenstoffatomen sind und

m und n die oben angegebene Bedeutung besitzen;

C) Verbindungen mit der Formel:

$$C—CHOH—SO_3M,$$

in der R ein Wasserstoffatom oder ein Hydroxymethylrest ist und M ein Alkalimetall oder eine Ammoniumgruppe ist, wobei aber ausgeschlossen sind:

a) 0,6 M injizierbare wässrige Lösungen von Ethanolaminbisulfit und
b) Zusammensetzungen, die ein Formaldehydbisulfit und ein Protamin enthalten.

2. Verfahren nach Anspruch 1, dadurch gekennzeichnet, daß das obige Behandlungsmittel eine

Verbindung des Typs (A) ist, in der $R_1$, $R_2$ und $R_3$ jeweils eine Alkylgruppe mit 1 bis 4 Kohlenstoffatomen bedeuten.

3. Verfahren nach Anspruch 2, dadurch gekennzeichnet, daß $R_1$, $R_2$ und $R_3$ jeweils Ethylreste bedeuten.

4. Verfahren nach Anspruch 1, dadurch gekennzeichnet, daß das aktive Behandlungsmittel eine Verbindung des Typs (A) ist, in der $R_1$ eine Alkylgruppe mit 5 bis 10 Kohlenstoffatomen und $R_2$ und $R_3$ beide Wasserstoff bedeuten.

5. Verfahren nach Anspruch 4, dadurch gekennzeichnet, daß $R_1$ einen n-Octylrest bedeutet.

6. Verfahren nach Anspruch 1, dadurch gekennzeichnet, daß das aktive Behandlungsmittel eine Verbindung des Typs (B) ist, in der $R_5$, $R_6$, $R_7$ und $R_8$ jeweils eine Alkylgruppe mit 1 bis 4 Kohlenstoffatomen bedeuten.

7. Verfahren nach Anspruch 6, dadurch gekennzeichnet, daß $R_5$, $R_6$, $R_7$ und $R_8$ jeweils einen Butylrest bedeuten.

8. Verfahren nach Anspruch 1, dadurch gekennzeichnet, daß das aktive Behandlungsmittel Natriumformaldehydbisulfit ist.

9. Verfahren nach einem der vorangehenden Ansprüche, dadurch gekennzeichnet, daß das aktive Behandlungsmittel ein Bisulfit ist.

10. Verfahren nach einem der vorangehenden Ansprüche, dadurch gekennzeichnet, daß die Zusammensetzung in einer oral verabreichbaren Form vorliegt.

11. Verfahren nach Anspruch 10, dadurch gekennzeichnet, daß die Zusammensetzung eine wässrige Lösung ist, die 1 bis 10 Gew.% des aktiven Behandlungsmittels enthält.

12. Verfahren nach einem der Ansprüche 1 bis 9, dadurch gekennzeichnet, daß die Zusammensetzung in injizierbarer Form vorliegt.

13. Verfahren nach Anspruch 12, dadurch gekennzeichnet, daß die Zusammensetzung eine injizierbare wässrige Lösung ist, die 1 bis 11 Gew.% des aktiven Behandlungsmittels enthält.

14. Verfahren nach einem der vorangehenden Ansprüche, dadurch gekennzeichnet, daß das aktive Behandlungsmittel der einzige pharmakologisch aktive Wirkstoff in der Zusammensetzung ist.

15. Verfahren nach einem der Ansprüche 1 bis 13, dadurch gekennzeichnet, daß die Zusammensetzung in Form einer wässrigen Lösung vorliegt, die gelöstes Schwefeldioxid enthält.

**Revendications pour les Etats Contractants: BE CH DE FR GB IT LI LU NL SE**

1. Composition pharmaceutique utilisable dans le traitement d'un état thrombotique établi ou débutant chez un mammifère, caractérisée en ce qu'elle contient au moins une substance servant d'excipient et, en tant qu'agent actif de traitement, au moins un composé choisi parmi les suivants:

(A) Composés de formule

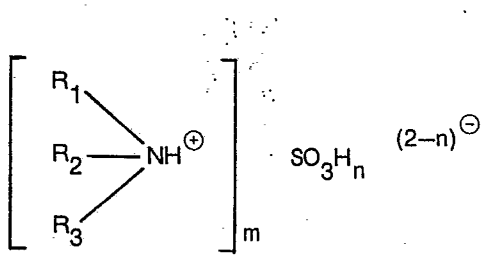

dans laquelle:

$R_1$ est un radical alcoyle contenant moins de 11 atomes de carbone, un radical cycloalcoyle contenant 6 à 10 atomes de carbone ou un radical monohydroxyalcoyle contenant moins de 11 atomes de carbone,

$R_2$ est un atome d'hydrogène, un radical alcoyle contenant moins de 11 atomes de carbone ou un radical monohydroxyalcoyle contenant moins de 11 atomes de carbone ou

$R_1$ et $R_2$ forment ensemble un noyau morpholinique, pipéridinique ou hexaméthylène-iminique non substitué ou l'un de ces noyaux portant, sur l'un de ses atomes de carbone, un radical alcoyle contenant moins de 11 atomes de carbone,

$R_3$ est un atome d'hydrogène, un radical alcoyle contenant moins de 11 atomes de carbone, un radical monohydroxyalcoyle contenant moins de 11 atomes de carbone ou un radical de formule

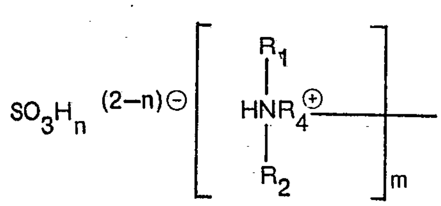

dans laquelle $R_1$ et $R_2$ ont les significations données ci-dessus, $R_4$ est un radical aliphatique saturé bivalent contenant moins de 11 atomes de carbone, et

m est égal à 1 ou 2, n est égal à 0 ou 1 et la somme de m et de n est égale à 2;

(B) Composés de l'une ou l'autre des formules

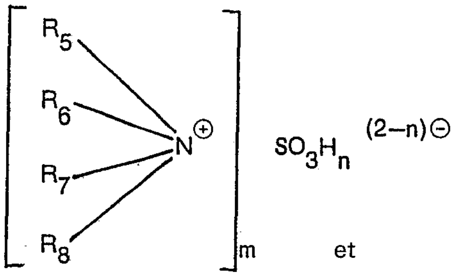

et

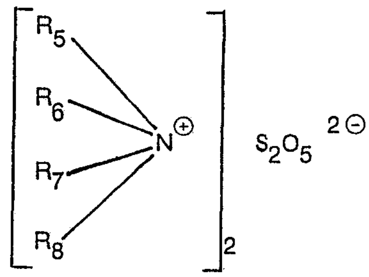

dans laquelle:

$R_5$, $R_6$, $R_7$ et $R_8$ sont semblables ou différents et représentent chacun un radical alcoyle contenant moins de 11 atomes de carbone et

m et n ont les significations données ci-dessus;

(C) Composés de formule

$$R—CHOH—SO_3M$$

dans laquelle:

R est un atome d'hydrogène ou un radical hydroxyméthyle et

M est un métal alcalin ou un groupe ammonium; mais à l'exclusion de:

(a) solutions aqueuses injectables 0,6M de bisulfite d'éthanolamine

(b) compositions contenant un bisulfite de formaldéhyde et une protamine.

2. Composition pharmaceutique selon la revendication 1, caractérisée en ce que l'agent actif de traitement est un composé de type (A) dans lequel $R_1$, $R_2$ et $R_3$ représentent chacun un groupe alcoyle contenant 1 à 4 atomes de carbone.

3. Composition pharmaceutique selon la revendication 2, caractérisée en ce que $R_1$, $R_2$ et $R_3$ représentent chacun un éthyle.

4. Composition pharmaceutique selon la revendication 1, caractérisée en ce que l'agent actif de traitement est un composé de type (A) dans lequel $R_1$ représente un groupe alcoyle contenant 5 à 10 atomes de carbone et $R_2$ et $R_3$ représentent chacun un hydrogène.

5. Composition pharmaceutique selon la revendication 4, caractérisée en ce que $R_1$ représente un n-octyle.

6. Composition pharmaceutique selon la revendication 1, caractérisée en ce que l'agent actif de traitement est un composé de type (B) dans lequel $R_5$, $R_6$, $R_7$ et $R_8$ représentent chacun un groupe alcoyle contenant 1 à 4 atomes de carbone.

7. Composition pharmaceutique selon la revendication 6, caractérisée en ce que $R_5$, $R_6$, $R_7$ et $R_8$ représentent chacun un butyle.

8. Composition pharmaceutique selon la revendication 1, caractérisée en ce que l'agent actif de traitement est le bisulfite de sodium-formaldéhyde.

9. Composition pharmaceutique selon l'une quelconque des revendications 1 à 8, caractérisée en ce que l'agent actif de traitement est un bisulfite.

10. Composition pharmaceutique selon l'une quelconque des revendications 1 à 9, caractérisée en ce qu'elle se présente sous une forme administrable oralement.

11. Composition pharmaceutique selon la revendication 10, caractérisée en ce qu'elle se présente sous la forme d'une solution aqueuse contenant 1 à 10% en poids de l'agent actif de traitement.

12. Composition pharmaceutique selon l'une quelconque des revendications 1 à 9, caractérisée en ce qu'elle se présente sous une forme injectable.

13. Composition pharmaceutique selon la revendication 12, caractérisée en ce qu'elle se présente sous la forme d'une solution aqueuse injectable contenant 1 à 11% en poids de l'agent actif de traitement.

14. Composition pharmaceutique selon l'une quelconque des revendications 1 à 13, caractérisée en ce que l'agent actif de traitement est le seul agent pharmacologiquement actif dans la composition.

15. Composition pharmaceutique selon l'une quelconque des revendications 1 à 13, caractérisée

en ce qu'elle se présente sous la forme d'une solution aqueuse contenant de l'anhydride sulfureux dissous.

16. Composition pharmaceutique selon l'une quelconque des revendications 1 à 9, utilisable dans le traitement d'un état thrombotique établi ou débutant chez l'homme ou un autre mammifère.

**Revendications pour l'Etat Contractant: AT**

1. Procédé pour la préparation d'une composition pharmaceutique utilisable dans le traitement d'un état thrombotique établi ou débutant chez un mammifère, caractérisé en ce qu'il consiste à mélanger au moins une substance servant d'excipient avec, en tant qu'agent actif de traitement, au moins un composé choisi parmi les suivants:

(A) Composés de formule

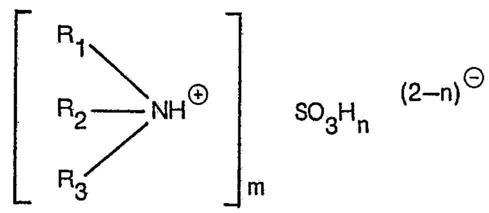

dans laquelle:

$R_1$ est un radical alcoyle contenant moins de 11 atomes de carbone, un radical cycloalcoyle contenant 6 à 10 atomes de carbone ou un radical monohydroxyalcoyle contenant moins de 11 atomes de carbone,

$R_2$ est un atome d'hydrogène, un radical alcoyle contenant moins de 11 atomes de carbone ou un radical monohydroxyalcoyle contenant moins de 11 atomes de carbone, ou

$R_1$ et $R_2$ forment ensemble un noyau morpholinique, pipéridinique ou hexaméthylène-iminique non substitué ou l'un de ces noyaux portant, sur l'un de ses atomes de carbone, un radical alcoyle contenant moins de 11 atomes de carbone,

$R_3$ est un atome d'hydrogène, un radical alcoyle contenant moins de 11 atomes de carbone, un radical monohydroxyalcoyle contenant moins de 11 atomes de carbone ou un radical de formule

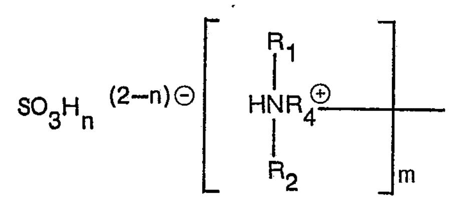

dans laquelle $R_1$ et $R_2$ ont les significations données ci-dessus et

$R_4$ est un radical aliphatique saturé bivalent contenant moins de 11 atomes de carbone;

m est égal à 1 ou 2, n est égal à 0 ou 1 et la somme de m et n est égale à 2;

(B) Composés de l'une ou l'autre des formules

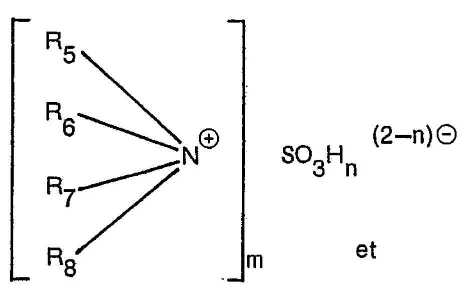

et

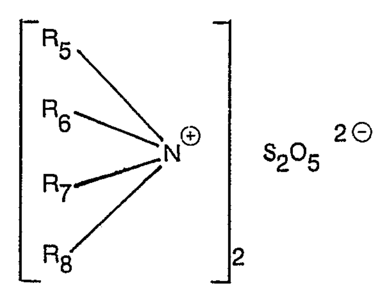

dans lesquelles

$R_5$, $R_6$, $R_7$ et $R_8$ sont semblables ou différents et représentent chacun un radical alcoyle contenant moins de 11 atomes de carbone et

m et n ont les significations données ci-dessus;

(C) Composés de formule

$$R—CHOH—SO_3M$$

dans laquelle

R est un atome d'hydogène ou un radical hydroxyméthyle et

M est un métal alcalin ou un groupe ammonium; mais à l'exclusion

(a) de solution aqueuses injectables 0,6M de bisulfite d'éthanolamine et
(b) de compositions contenant un bisulfite de formaldéhyde et une protamine.

2. Procédé selon la revendication 1, caractérisé en ce que l'agent actif de traitement est un composé de type (A) dans lequel $R_1$, $R_2$ et $R_3$ représentent chacun un groupe alcoyle contenant 1 à 4 atomes de carbone.

3. Procédé selon la revendication 2, caractérisé en ce que $R_1$, $R_2$ et $R_3$ représentent chacun un éthyle.

4. Procédé selon la revendication 1, caractérisé en ce que l'agent actif de traitement est un composé de type (A) dans lequel $R_1$ représente un groupe alcoyle contenant 5 à 10 atomes de carbone et $R_2$ et $R_3$ représentent chacun un hydrogène.

5. Procédé selon la revendication 4, caractérisé en ce que $R_1$ représente un n-octyle.

6. Procédé selon la revendication 1, caractérisé en ce que l'agent actif de traitement est un composé de type (B) dans lequel $R_5$, $R_6$, $R_7$ et $R_8$ représentent chacun un groupe alcoyle contenant 1 à 4 atomes de carbone.

7. Procédé selon la revendication 6, caractérisé en ce que $R_5$, $R_6$, $R_7$ et $R_8$ représentant chacun un butyle.

8. Procédé selon la revendication 1, caractérisé en ce que l'agent actif de traitement est le bisulfite de sodium-formaldéhyde.

9. Procédé selon l'une quelconque des revendications 1 à 8, caractérisé en ce que l'agent actif de traitement est un bisulfite.

10. Procédé selon l'une quelconque des revendications 1 à 9, caractérisé en ce que la composition se présente sous une forme administrable oralement.

11. Procédé selon la revendication 10, caractérisé en ce que la composition est une solution aqueuse contenant 1 à 10% en poids de l'agent actif de traitement.

12. Procédé selon l'une quelconque des revendications 1 à 9, caractérisé en ce que la composition se présente sous une forme injectable.

13. Procédé selon la revendication 12, caractérisé en ce que la composition est une solution aqueuse injectable contenant 1 à 11% en poids de l'agent actif de traitement.

14. Procédé selon l'une quelconque des revendications 1 à 13, caractérisé en ce que l'agent actif de traitement est le seul agent pharmaceutiquement actif dans la composition.

15. Procédé selon l'une quelconque des revendications 1 à 13, caractérisé en ce que la composition se présente sous la forme d'une solution aqueuse contenant de l'anhydryde sulfureux dissous.